(19) Europäisches Patentamt
European Patent Office
Office européen des brevets

(11) EP 1 622 624 B1

(12) **EUROPEAN PATENT SPECIFICATION**

(45) Date of publication and mention of the grant of the patent:
**26.12.2007 Bulletin 2007/52**

(21) Application number: **04730660.0**

(22) Date of filing: **30.04.2004**

(51) Int Cl.:
***A61K 31/635*** *(2006.01)* ***A61K 31/555*** *(2006.01)*
***A61K 31/505*** *(2006.01)* ***A61K 31/7072*** *(2006.01)*
***A61P 35/00*** *(2006.01)*

(86) International application number:
**PCT/JP2004/006292**

(87) International publication number:
**WO 2004/098613 (18.11.2004 Gazette 2004/47)**

(54) **MEDICINAL COMPOSITIONS COMPRISING N-(3-CHLORO-1H-INDOL-7-YL)-4-SULFAMOYLBENZENESULFONAMIDE AND FURTHER CYTOSTATICS**

MEDIZINISCHE ZUSAMMENSETZUNGEN ENTHALTEND N-(3-CHLORO-1H-INDOL-7-YL)-4-SULFAMOYLBENZENSULFONAMID UND WEITERE CYTOSTATIKA

COMPOSITIONS MEDICINALES CONTENANT N-(3-CHLORO-1H-INDOL-7-YL)-4-SULFAMOYLBENZENESULFONAMIDE ET AUTRES CYTOSTATIQUES

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **08.05.2003 US 431569**

(43) Date of publication of application:
**08.02.2006 Bulletin 2006/06**

(73) Proprietor: **Eisai R&D Management Co., Ltd.**
**Tokyo 112-8088 (JP)**

(72) Inventor: **OZAWA, Yoichi**
**Tsukuba-shi, Ibaraki (JP)**

(74) Representative: **HOFFMANN EITLE**
**Patent- und Rechtsanwälte**
**Arabellastrasse 4**
**81925 München (DE)**

(56) References cited:
**WO-A-02/36117**

EP 1 622 624 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

Technical Field

The present invention relates to a novel medicinal composition comprising a compound useful as an anticancer drug, particularly N-(3-chloro-1H-indol-7-yl)-4-sulfamoylbenzenesulfonamide or a pharmacologically acceptable salt thereof, combined with another anticancer agent.

Prior Art

As chemicals used conventionally as chemotherapeutic drugs for cancers, there are a large number of chemicals such as cyclophosphamide as an alkylating agent, methotrexate and fluorouracil as antimetabolites, doxorubicin, mitomycin and bleomycin as antibiotics, vincristine and etoposide derived from plant, and cisplatin as a metal complex, but their antitumor effects are insufficient, so there is a demand for development of new antitumor agents.

On one hand, the present inventors succeeded for the first time in synthesis of completely new aromatic sulfonamides or aromaticsulfonate compounds,they unexpectedlyfound that these compounds exhibit an excellent antitumor activity, and they provided useful antitumor agents showing new mechanism of action (JP-A 7-165708). In particular, N-(3-chloro-1H-indol-7-yl)-4-sulfamoylbenzenesulfonamide (hereinafter, also called "E7070") represented by the following formula:

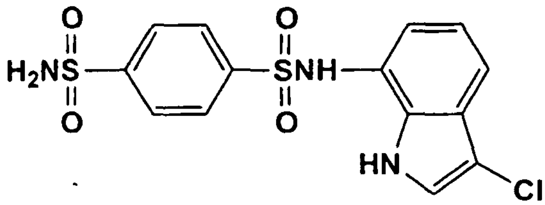

exhibits an activity on various types of tumors and is very useful.

There is the case where a certain kind of compound is effective against a certain kind of cancer but poor in effectiveness against other cancers. Further, administration of a single component may not achieve a sufficient effect. That is, the object of the present invention is to provide a medicinal composition having an excellent antitumor activity capable of solving these problems.

Disclosure of the Invention

In view of the foregoing, the present inventors made extensive study to search for an excellent antitumor composition, and as a result, they unexpectedly found that a synergistically further excellent antitumor activity can be achieved by using a certain anticancer agent in combination with N-(3-chloro-1H-indol-7-yl)-4-sulfamoylbenzenesulfonamide or a salt thereof, and the present invention was thereby completed.

That is, the present invention relates to:

1) a medicinal composition comprising N-(3-chloro-1H-indol-7-yl)-4-sulfamoylbenzenesulfonamide or a salt thereof, combined with and at least one substance selected from (1) carboplatin; (2) oxaliplatin; and (3) a salt of the above-mentioned (1) to (2);
2) the medicinal composition described in the above 1), which is an anticancer agent;
3) the medicinal composition described in the above 2), which comprises N-(3-chloro-1H-indol-7-yl)-4-sulfamoylbenzenesulfonamide or a salt thereof, and carboplatin or a salt thereof;
4) the medicinal composition described in the above 2), which comprises N-(3-chloro-1H-indol-7-yl)-4-sulfamoylbenzenesulfonamide or a salt thereof, and oxaliplatin or a salt thereof;
5) the medicinal composition described in the above 4, which further comprises 5-fluorouracil or a salt thereof;
6) the medicinal composition described in the above 5), which is an anticancer agent;
7) use of N-(3-chloro-1H-indol-7-yl)-4-sulfamoylbenzenesulfonamide or a salt thereof, and at least one substance selected from (1) carboplatin; (2) oxaliplatin; and (3) a salt of the above-mentioned (1) to (2), for producing an agent for preventing or treating a cancer, as further defined in the claims;
8) use of N-(3-chloro-1H-indol-7-yl)-4-sulfamoylbenzenesulfonamide or a salt thereof, 5-fluorouracil or a salt thereof,

and oxaliplatin or a salt thereof, for producing an agent for preventing or treating a cancer;

9) a pharmaceutical kit for administering effective amounts of N-(3-chloro-1H-indol-7-yl)-4-sulfamoylbenzenesulfonamide or a salt thereof and oxaliplatin or a salt thereof simultaneously or separately to a patient; and

10) the pharmaceutical kit described in the above 9), which further comprises 5-fluorouracil or a salt thereof.

Hereinafter, the meanings of symbols, terms etc. used in this specification are described, and the present invention is described in detail.

In the specification of this application, although structural formula of a compound may express a certain isomer for the sake of convenience, it goes without saying that the compounds contained in the medicinal composition according to the present invention are not limited to those of the structural formulae shown for convenience's sake and all possible isomers which can occur in the structures, for example geometric isomer, optical isomer based on asymmetrical carbon, rotational isomer, stereoisomer and tautomer, as well as a mixture of such isomers are included. Further, as the above-mentioned compound, crystal polymorphism may be present but, again, there is no limitation but any of single crystal form or a mixture will do. Further, the above-mentioned compound may be an anhydride or a hydrate, and either of them are included in the scope of claim for patent in the present invention. The medicinal composition according to the present invention exhibits a strong antitumor activity, and it also includes derivatives of the above-mentioned, which exhibit an antitumor activity as a result of metabolism such as oxidation, reduction, hydrolysis and conjugation in vivo. Further, the present invention also includes the compounds which produce the compound in the composition of the present invention as a result of metabolism such as oxidation, reduction and hydrolysis in vivo.

In this specification, N-(3-chloro-1H-indol-7-yl)-4-sulfamoylbenzenesulfonamide and E7070 refer to a compound represented by the formula:

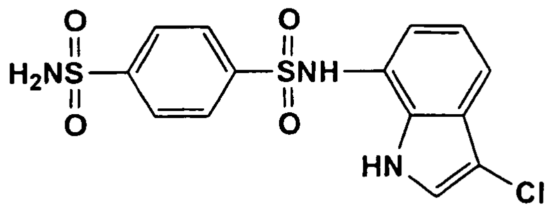

5-Fluorouracil (5-FU (5-fluoro-2,4-(1H,3H)-pyrimidinedione)) in this specification refers to a compound represented by the formula:

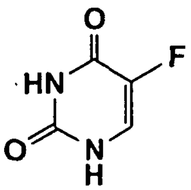

Carboplatin (cis-diammine (1,1-cyclobutanedicarboxylato)platinum) in this specification refers to a compound represented by the formula:

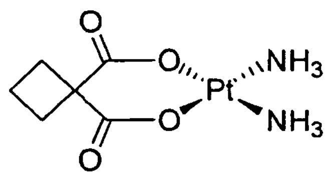

Oxaliplatin (oxalato(1R, 2R-cyclohexanediamine)platinum) in this specification refers to a compound represented by the formula:

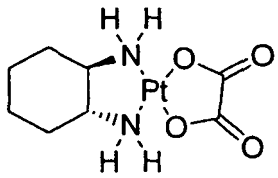

Capecitabine (N-4-pentyloxycarbonyl-5'-deoxy-5-fluorocytidine) in this specification refers to a compound represented by the formula:

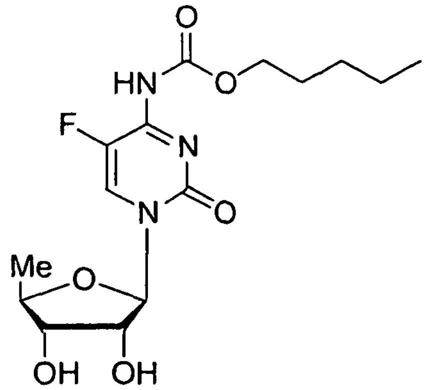

The type of the "salts" used in this specification is not particularly limited, and examples thereof include additive salts of inorganic acids such as hydrochloride, sulfate, carbonate, bicarbonate, hydrobromate, hydroiodate etc.; additive salts of organic carboxylic acids such as acetate, maleate, lactate, tartarate, trifluoroacetate etc.; additive salts of organic sulfonic acid such as methanesulfonate, hydroxymethanesulfonate, hydroxyethanesulfonate, benzenesulfonate, toluenesulfonate, taurine salt etc.; additive salts of amines such as trimethylamine salt, triethylamine salt, pyridine salt, procaine salt, picoline salt, dicyclohexylamine salt, N,N'-dibenzylethylene diamine salt, N-methylglucamine salt, diethanolamine salt, triethanolamine salt, tris(hydroymethylamino)methane salt, phenethylbenzylamine salt etc.; and additive salts of amino acids such as arginine salt, lysine salt, serine salt, glycine salt, aspartate, glutamate, etc.

N-(3-Chloro-1H-indol-7-yl)-4-sulfamoylbenzenesulfonamide (E7070) can be synthesized according to a known method (WO95/07276 or Example 19 in JP-A 7-165708) or its analogous method.

5-Fluorouracil, carboplatin and oxaliplatin are known compounds and can be produced in known methods or their analogous methods.

The starting compound and various reagents in the production processes may have formed salts or hydrates which vary depending on the starting material, the solvent used etc., and are not particularly limited so long as the reaction is not inhibited. Also, the solvent used varies depending on the starting material, reagents etc., and is not particularly limited insofar as it is inert to the reaction and dissolves the starting material to a certain degree. When the desired compound is obtained in a free form, it can be converted in a usual manner into a pharmaceutically acceptable salt. Further, the resultant various isomers (for example, geometric isomer, optical isomer based on asymmetric carbon, rotational isomer, stereoisomer, tautomer, etc.) can be purified and isolated by usual separating means, for example, re-crystallization, diastereomer salt method, enzyme resolution method, and various kinds of chromatography (for example, thin-layer chromatography, column chromatography, gas chromatography, etc.).

The "anticancer drug" in this specification means a drug used against tumors, particularly malignant tumors. The cancer disease against which the medicinal composition or the anticancer drug according to the present invention is effective includes, for example, brain cancer, head and neck cancer, cancer of the esophagus, cancer of the stomach, cancer of the colon, hepatoma, pancreatic cancer, lung cancer, breast cancer, skin cancer, ovarian cancer, prostatic cancer, renal cancer, bladder cancer, lymphoma, leukemia, etc. The medicinal composition or the anticancer drug according to the present invention is useful for treating or preventing cancer diseases in mammalians (e. g. , humans, mice, rats, guinea pigs, rabbits, dogs, horses, monkeys, etc.), particularly for treating or preventing cancer diseases in humans.

In the present invention, "the medicinal composition ..., combined with" and "the medicinal composition comprising" refer respectively to a "medicinal composition" comprising two or more active ingredients or anticancer agents combined therein, and the "medicinal composition" is prepared and administered as a single preparation comprising two or more active ingredients. Specifically, the synergistic effect can be increased

(A) by a method for preventing or treating cancers, by administering E7070 or a salt thereof to a patient; and administering, after a predetermined time, at least one substance selected from (1) carboplatin; (2) oxaliplatin; and (3) a salt of the above-mentioned (1) to (2) to the patient,

(B) by a method for preventing or treating cancers, by administering E7070 or a salt thereof to a patient; and administering, after a predetermined time, 5-fluorouracil or a salt thereof, and oxaliplatin or a salt thereof to the patient,

(C) by a method for preventing or treating cancers, by administering at least one substance selected from oxaliplatin; and a salt thereof to a patient; and administering, after a predetermined time, E7070 or a salt thereof to the patient, and the present invention encompasses such methods for preventing or treating, or

(D) by a method for preventing or treating cancers, by administering 5-fluorouracil or a salt thereof, and oxaliplatin or a salt thereof to a patient; and administering, after a predetermined time, E7070 or a salt thereof to the patient, and the present invention encompasses such methods for preventing or treating.

The synergistic antitumor effect can also be achieved by using

(A) a pharmaceutical kit for administering effective amounts of E7070 or a salt thereof and at least one substance selected from oxaliplatin; and a salt thereof simultaneously or separately to a patient, for the purpose of administration of the respective ingredients at predetermined intervals as in the above-mentioned methods for preventing or treating, or

(B) a pharmaceutical kit for administering effective amounts of E7070 or a salt thereof, 5-fluorouracil or a salt thereof, and oxaliplatin or a salt thereof simultaneously or separately to a patient, for the purpose of administration of the respective ingredients at predetermined intervals as in the above-mentioned methods for preventing or treating. The present invention encompasses such a pharmaceutical kit. For example, mention is made of a pharmaceutical kit wherein the ingredients, each being packed in a small vessel, are packed in one box.

When the medicinal composition of the present invention is used as a pharmaceutical preparation, the administration form is not particularly limited, and it is administered orally or parenterally. It is useful for treatment and prevention in mammalians (e.g., humans, mice, rats, guineapigs, rabbits, dogs, horses, monkeys, etc.), particularly in humans. The administration dose varies depending on the severity of symptoms, the age, sex, body weight and chemical sensitivity of the patient, administration form, administration time, administration intervals, the properties, prescription and type of the pharmaceutical preparation, and the type of active ingredient, and is not particularly limited. For example, E7070 or a salt thereof is given daily in one portion or in divided portions into a man in a dose of usually about 5 to 6000 mg, preferably about 50 to 4000 mg, more preferably 100 to 3000 mg. The daily doses of the other ingredients are usually as follows: 5-fluorouracil, 5 to 20 mg/kg; carboplatin, 300 to 400 mg/$m^2$; and oxaliplatin, 85 to 130 mg/$m^2$, but these are standard doses in the case of administering a single drug. In the present invention, the dose can be suitably changed depending on the constitution of the ingredient. For example, the daily dose of the respective ingredients for an adult can be determined usually in the range of 1 to 6000 mg, preferably about 10 to 1000 mg, more preferably 20 to 300 mg.

The medicinal composition according to the invention can be prepared by using the active ingredients as they are or mixing them with pharmacologically acceptable carriers known per se by a conventional method. Preferable preparation forms include injections, tablets, powders, fine granules, granules, coated tablets, capsules, syrups, troches, inhalations, suppositories, ointments, eye ointments, eye drops, nose drops, ear drops, poultices and lotions. To prepare these pharmaceutical preparations, ordinarily used fillers, binders, lubricants, coloring agents, flavoring agents and, if necessary, stabilizers, emulsifiers, absorption promoters and surfactants can be used. Ingredients used generally as starting materials for pharmaceutical preparations can be blended in a usual manner for manufacturing.

As the above-mentioned preparation components, for example, include animal or vegetable oils such as soybean oil, beef tallow or synthetic glyceride; hydrocarbons such as liquid paraffin, squalane or solid paraffin; ester oils such as octyldodecyl myristate or isopropyl myristate; higher alcohols such as cetostearyl alcohol or behenyl alcohol; silicon resin; silicon oil; surfactants such as polyoxyethylene fatty acid ester, sorbitan fatty acid ester, glycerol fatty acid ester, polyoxyethylenesorbitan fatty acid ester, polyoxyethylene hydrogenated castor oil or polyoxyethylene/polyoxypropylene block copolymers; water-soluble polymers such as hydroxyethyl cellulose, polyacrylic acid, carboxyvinyl polymers, polyethylene glycol, polyvinylpyrrolidone or methyl cellulose; alcohols such as ethanol or isopropanol; polyhydric alcohols such as glycerol, propylene glycol, dipropylene glycol or sorbitol; sugars such as glucose or sucrose; inorganic powders such as silicic anhydride, aluminum magnesium silicate or aluminum silicate; and purified water. For pH adjustment, inorganic acids such as hydrochloric acid or phosphoric acid, alkali metal salts of inorganic acids such as sodium phosphate, inorganic bases such as sodium hydroxide, organic acids such as lower fatty acids, citric acid or lactic acid, alkali metal salts of organic acids such as sodium citrate or sodium lactate, organic bases such as arginine or ethanolamine, etc. can be used. If necessary, a preservative, an antioxidant, etc. can be added.

For example, when a solid preparation for oral administration is prepared, filler and, if necessary, binder, disintegrating agent, lubricant, coloring agent, flavoring agent etc. are added to the main ingredient, followed by subjecting

to a common method to make into tablets, coated tablets, granules, fine granules, powders, capsules etc.

Examples of the filler are lactose, corn starch, sucrose, glucose, sorbitol, crystalline cellulose and silicon dioxide; examples of the binder are polyvinyl alcohol, ethyl cellulose, methyl cellulose, gum arabic, hydroxypropyl cellulose and hydroxypropyl methyl cellulose; examples of the lubricant are magnesium stearate, talc and silica; examples of the coloring agent are those which are allowed to add to pharmaceuticals; and examples of the flavoring agents are cacao powder, menthol, aromatic, peppermint oil, borneol and cinnamon powder. It is of course no problem that such tablets and granules are appropriately coated with a sugar coat, gelatin coat or others if necessary.

In preparing injections, a pH adjusting agent, a buffer, a suspending agent, a solubilizer, a stabilizer, an isotonizing agent, a preservative etc. are added, if necessary, to the main ingredient, followed by subjecting to a conventional method to make into injections for intravenous, subcutaneous or intramuscular administration. At that time, it may be made into a freeze-dried product by a common method if necessary.

Examples of the suspending agent are methyl cellulose, polysorbate 80, hydroxyethyl cellulose, gum arabic, tragacanth powder, sodium carboxymethyl cellulose and polyoxyethylene sorbitan monolaurate.

Examples of the solubilizer are polyoxyethylene hydrogenated castor oil, polysorbate 80, nicotinamide, polyoxyethylene sorbitan monolaurate, macrogol and castor oil fatty acid ethyl ester.

Examples of the stabilizer are sodium sulfite and sodium metasulfite. Examples of the preservative are methyl para-hydroxybenzoate, ethyl para-hydroxybenzoate, sorbic acid, phenol, cresol and chlorocresol.

According to the present invention, there can be provided a novel combined medicinal composition exhibiting an antitumor activity even on cancers against which conventional anticancer drugs are not sufficiently effective. The medicinal composition according to the present invention is useful for treating or preventing brain cancer, head and neck cancer, cancer of the esophagus, cancer of the stomach, cancer of the colon, hepatoma, pancreatic cancer, lung cancer, breast cancer, skin cancer, ovarian cancer, prostatic cancer, renal cancer, bladder cancer, lymphoma, leukemia, etc. Administration of smaller amounts of the combined active ingredients in the composition of the present invention, as compared with administration of large amounts of each single ingredient, hardly causes the side effect of each ingredient, can reduce the side effects of the whole ingredients, and can reduce the cost burden which is caused by using a large amount of an expensive drug for a long time, by simultaneously using inexpensive drugs having a relatively strong effect.

## Brief Description of the Drawings

Fig. 1 is a graph showing the effect of the combination of E7070 and capecitabine. The relative tumor weight (above) and the relative body weight (below) are shown on the ordinate, and the number of days after administration was initiated is shown on the axis.

Fig. 2 is a graph showing the criteria of the isobologram. The dose of B is shown on the ordinate, and the dose of A is shown on the axis.

Fig. 3 is a graph showing the criteria of the isobologram analysis of the combination of E7070 with cisplatin in HCT15 cells. The dose of cisplatin is shown on the ordinate, and the dose of E7070 is shown on the axis.

Fig. 4 is a graph showing the criteria of the isobologram analysis of the combination of E7070 with carboplatin in HCT15 cells. The dose of carboplatin is shown on the ordinate, and the dose of E7070 is shown on the axis.

Fig. 5 is a graph showing the criteria of the isobologram analysis of the combination of E7070 with oxaliplatin in HCT15 cells. The dose of oxaliplatin is shown on the ordinate, and the dose of E7070 is shown on the axis.

## Examples

Hereinafter, Examples and Test Examples are described to show the advantageous effect of the composition of the present invention, but these are illustrative, and in any case the present invention is not limited to the following specific examples.

As described above, the medicinal composition comprising E7070 or a salt thereof combined with at least one substance selected from (1) carboplatin; (2) oxaliplatin; (3) capecitabine; and (4) a salt of the above-mentioned (1) to (3) and the medicinal composition comprising E7070 or a salt thereof, 5-fluorouracil or a salt thereof and oxaliplatin or a salt thereof exhibit an excellent antitumor activity due to the synergistic antitumor effect of each single drug, and the presence or absence of this synergy was examined according to a two-way ANOVA method (see the following literatures (i) to (iii): (i)Effects of 5-fluorouracil, leucovorin, and glucarate in rat colon-tumor explants. Cancer Chemother Pharmacol. 1992;30(1):25-30; (ii)Enhancement of vincristine cytotoxicity in drug-resistant cells by simultaneous treatment with onconase, an antitumor ribonuclease. J Natl Cancer Inst. 1996 Jun 5;88(11):747-53; (iii) Effects of growth hormone and testosterone on cortical bone formation and bone density in aged orchiectomized rats. Bone. 1999 May;24(5):491-7).

Example 1 Combined use of E7070 and capecitabine in human breast cancer HBC4 xenograft model (Reference Example)

Human breast cancer HBC4 (purchased from the Cancer Chemotherapy Center, Japan Foundation for Cancer Research (Tokyo, Japan)) was cultured in RPMI1640 (containing 10% FBS) in a 5% $CO_2$ gas incubator until it attained about 80% confluence, and the cells were harvested with trypsin-EDTA and suspended in Hanks balanced solution to prepare a suspension of $5\times10^7$ cells/ml. 0..1 ml of the cell suspension was implanted subcutaneously in each nude mouse. When the average tumor volume reached 114 $mm^3$ after the implantation, E7070 in a dose of 30. 625 mg/kg/day and/or capecitabine in a dose of 1.3125 mmol/kg/day were administered either alone or in combination. E7070 alone was intravenously administered once per day for 5 days (first to fifth days), while capecitabine alone was intravenously administered once per day for 14 days (first to fourteenth days). Previous administration of E7070 (E7070 on the first to fifth days; capecitabine on the sixth to nineteenth days) was performed. After the administration was initiated, the longer and shorter dianometers of the tumor were measured at the frequency of 2 times/week with digital calipers (Mitsutoyo), and the tumor volume was calculated according to the following equation.

$$\text{Tumor volume} = \text{longer diameter of tumor (mm)} \times \text{shorter diameter of tumor (mm)}^2/2$$

The items for determination of antitumor effect were the following 2 items:

$T_{x4}$: The time in days for tumor size to attain 4 folds of initial size.
Minimum relative tumor volume (mRTV) : Minimum value of relative tumor volume (RTV*)
*: Tumor volume on n days after first treatment/tumor volume at the start of treatment.

When the group treated with the combined drugs exhibited an antitumor effect superior to that of the group treated with the single drug and simultaneously a statistically significant interaction was recognized in two-way ANOVA, the antitumor effect was determined to be synergistic. Results are as shown in Table 1 and Fig. 1.

As is evident from Table 1 and Fig. 1, combined use of E7070 and capecitabine permits their respective effects to be additively increased, indicating that a combined drug of E7070 and capecitabine serves as an excellent anticancer agent.

Example 2 Evaluation of the effect of combined use of E7070, cisplatin*, carboplatin, and oxaliplatin in human colon cancer HCT15 cells in vitro (*: Reference Example)

Rapidly growing Human colon cancer strain HCT15 cells(purchased from ATCC) were harvested, counted, and inoculated at appropriate concentrations (1,250 cells/well) into 96-well microplates. The following day (day0), drugs were dissolved in DMSO(E7070), saline (cisplatin and carboplatin) or 5% glucose solution (oxaliplatin), diluted with culture medium at various concentrations, and then applied to triplicate culture wells.

In the case of simultaneous treatment, E7070 and cisplatin, carboplatin, or oxaliplatin were added at a time. After further 72-h incubation at 37°C and 5% CO2, the amount of viable cells was determined by the MTT assay (day3).

In the case of sequential treatment (E7070 pretreatment), E7070 was added on day 0. After 24-h incubation, E7070 was washed out with fresh medium and then platinum was added (day1). After another 48-h incubation at 37°C and 5% CO2, the amount of viable cells was determined by the MTT assay (day3).

In the case of sequential treatment (platinum pretreatment), platinum, that is, cisplatin, carboplatin or oxaliplatin was added on day 0. After 24-h incubation, platinum was washed out with fresh medium and then E7070 was added (day1). After another 48-h incubation at 37°C and 5% CO2, the amount of viable cells was determined by the MTT assay (day3).

The effects of E7070 in combination with cisplatin, carboplatin or oxaliplatin were analyzed using the isobologram method of Steel and Peckam (Steel GG and Peckham MJ: Exploitable mechanisms in combined radiotherapy-chemotherapy: the concept of additivity. Int J Radiat Oncol Biol Phys 5: 85-91, 1979), and modified by Kano et al (Kano Y, Suzuki K, Akutsu M, et al: Effects of CPT-11 in combination with other anti-cancer agents in culture. Int J Cancer 50: 604-610, 1992). The criteria of the isobologram are schematically shown in Fig. 2. Three lines (mode I, mode IIa and mode IIb) are IC50 values estimated from growth suppression curves of drug A alone and drug B alone. When the actual IC50 values in combination (data points) are in the area surrounded by these lines (envelope of additivity), that combination is evaluated as additive(point Pb). When the data points fell within the area of left side (point Pa), right side of the

envelope (point Pc), and outside the square, the interaction between two drugs was considered to be synergistic, antagonistic, and protective, respectively. Results of E7070 and platinum are as shown in Fig. 3, 4 and 5.

As is evident from Fig. 3, 4 and 5, combined use of E7070 and cisplatin demonstrated synergistic effect only in E7070 pre-treatment treatment, but not in other treatment schedules. On the other hand, the combinations of E7070 with carboplatin or oxaliplatin show synergistic effectinsimultaneous treatment. Furthermore, combined use of E7070 oxaliplatin permits their respective effects to be synergistically increased in oxaliplatin pre-treatment schedule. These data indicate that 1) the combination of E7070 with platinum is useful regimen in the aspect of enhancement of antitumor effect synergistically. 2) in the enhancement of antitumor effect, the combination of E7070 with carboplatin or oxaliplatin may have flexibility regarding treatment schedules, compared with that with cisplatin. That is, in the combination of E7070 with carboplatin may be admitted the simultaneous administration of both drugs, and E7070 pre-treatment. E7070 and oxaliplatin may be administered simultaneously, or one of the two may be administered after a predetermined time, and E7070 and oxaliplatin, whichever is administered first, exhibits a synergistic effect.

As is evident from the above-mentioned experimental examples, the compositions of the present invention exhibit an excellent antitumor activity and are useful as an antitumor agent. As the cancer type, cancers against which the respective ingredients are effective may be proposed, and since it varies depending on the constitution of the composition and further the synergistic effect in the present invention is higher than that of a single drug, the type of cancer is not particularly limited.

Table 1

Antitumor effect of single or combined drug of E7070 and capecitabine in HBC4 xenograft model

| Treatment | | | Antitumor effect | | | | | | |
|---|---|---|---|---|---|---|---|---|---|
| | E7070 (mg/kg/day) | Capecitabine (mmol/kg/day) | Tx4 (days) | T·C (days) | 2Way ANOVA | mRTV | 2Way ANOVA | PR | CR |
| Control | - | - | 22.0±3.8 | - | - | - | - | 0/5 | 0/5 |
| E7070 alone | 30.625(87.5%(MTD) | - | 50.0±8.1 | 28.0 | - | 0.24±0.07 | - | 6/6 | 0/6 |
| Capecitabine alone | - | 1.3125(87.5%MTD) | 29.0±2.5 | 7.0 | - | - | - | 0/6 | 0/6 |
| E7070+Capecitabine | 30.625 | 1.3125 | 51.2±16.4 | 29.2 | P=0.492 | 0.18±0.07 | P=0.009 | 6/6 | 1/6 |

m(minimum)RTV: RTV= tumor volume on n days after first treatment / tumor volume at the start of treatment. "." means no tumor regression.
m(minimum)RBW: RBW= body weight on n days after first treatment / body weight at the start of treatment
Partial response (PR): The incidence of tumor regression (more than 50% of tumor regression).
Complete response(CR): The incidence of tumor free mice
RBW<0.80: The incidence of more than 20% body weight loss (undue toxicity)

**Claims**

1. A medicinal composition comprising N-(3-chloro-1H-indol-7-yl)-4-sulfamoylbenzenesulfonamide or a salt thereof, combined with at least one substance selected from (1) carboplatin; (2) oxaliplatin; and (3) a salt of the above-mentioned (1) to (2).

2. The medicinal composition according to claim 1, which comprises N-(3-chloro-1H-indol-7-yl)-4-sulfamoylbenzenesulfonamide or a salt thereof, and carboplatin or a salt thereof.

3. The medicinal composition according to claim 1, which comprises N-(3-chloro-1H-indol-7-yl)-4-sulfamoylbenzenesulfonamide or a salt thereof, and oxaliplatin or a salt thereof.

4. The medicinal composition according to 3, which further comprises 5-fluorouracil or a salt thereof.

5. Use of N-(3-chloro-1H-indol-7-yl)-4-sulfamoylbenzenesulfonamide or a salt thereof, and oxaliplatin or a salt thereof, for producing an agent for preventing or treating cancer.

6. The use according to claim 5, wherein N-(3-chloro-1H-indol-7-yl)-4-sulfamoylbenzenesulfonamide or a salt thereof, and oxaliplatin or a salt thereof are to be administered simultaneously.

7. The use according to claim 5, wherein N-(3-chloro-1H-indol-7-yl)-4-sulfamoylbenzenesulfonamide or a salt thereof is to be administered, and oxaliplatin or a salt thereof is to be administered after a predetermined time.

8. The use according to claim 5, wherein oxaliplatin or a salt thereof is to be administered, and N-(3-chloro-1H-indol-7-yl)-4-sulfamoylbenzenesulfonamide or a salt thereof is to be administered after a predetermined time.

9. The use according to claim 5, wherein the agent further comprises 5-fluorouracil or a salt thereof.

10. Use of N-(3-chloro-1H-indol-7-yl)-4-sulfamoylbenzenesulfonamide or a salt thereof, and carboplatin or a salt thereof, for producing an agent for preventing or treating cancer, wherein N-(3-chloro-1H-indol-7-yl)-4-sulfamoylbenzenesulfonamide or a salt thereof, and carboplatin or a salt thereof are to be administered simultaneously.

11. Use of N-(3-chloro-1H-indol-7-yl)-4-sulfamoylbenzenesulfonamide or a salt thereof, and carboplatin or a salt thereof, for producing an agent for preventing or treating cancer, wherein N-(3-chloro-1H-indol-7-yl)-4-sulfamoylbenzenesulfonamide or a salt thereof is to be administered, and carboplatin or a salt thereof is to be administered after a predetermined time.

12. A pharmaceutical kit for administering effective amounts of N-(3-chloro-1H-indol-7-yl)-4-sulfamoylbenzenesulfonamide or a salt thereof, and oxaliplatin or a salt thereof, simultaneously or separately to a patient.

13. The pharmaceutical kit according to claim 12, which further comprises 5-fluorouracil or a salt thereof.

**Patentansprüche**

1. Medizinische Zusammensetzung, umfassend N-(3-Chloro-1H-indol-7-yl)-4-sulfamoylbenzolsulfonamid oder ein Salz davon, kombiniert mit mindestens einer Substanz, ausgewählt aus (1) Carboplatin; (2) Oxaliplatin; und (3) einem Salz der oben genannten (1) bis (2).

2. Medizinische Zusammensetzung gemäß Anspruch 1, welche N-(3-Chloro-1H-indol-7-yl)-4-sulfamoylbenzolsulfonamid oder ein Salz davon und Carboplatin oder ein Salz davon umfasst.

3. Medizinische Zusammensetzung gemäß Anspruch 1, welche N-(3-Chloro-1H-indol-7-yl)-4-sulfamoylbenzolsulfonamid oder ein Salz davon und Oxaliplatin oder ein Salz davon umfasst.

4. Medizinische Zusammensetzung gemäß 3, welche ferner 5-Fluorouracil oder ein Salz davon umfasst.

5. Verwendung von N-(3-Chloro-1H-indol-7-yl)-4-sulfamoylbenzolsulfonamid oder eines Salzes davon und von Oxaliplatin oder eines Salzes davon zur Herstellung eines Mittels zur Vorbeugung oder Behandlung von Krebs.

6. Verwendung gemäß Anspruch 5, worin N-(3-Chloro-1H-indol-7-yl)-4-sulfamoylbenzolsulfonamid oder ein Salz davon und Oxaliplatin oder ein Salz davon gleichzeitig zu verabreichen sind.

7. Verwendung gemäß Anspruch 5, worin N-(3-Chloro-1H-indol-7-yl)-4-sulfamoylbenzolsulfonamid oder ein Salz davon zu verabreichen ist und Oxaliplatin oder ein Salz davon nach einem vorbestimmten Zeitraum zu verabreichen ist.

8. Verwendung gemäß Anspruch 5, worin Oxaliplatin oder ein Salz davon zu verabreichen ist und N-(3-Chloro-1H-indol-7-yl)-4-sulfamoylbenzolsulfonamid oder ein Salz davon nach einem vorbestimmten Zeitraum zu verabreichen ist.

9. Verwendung gemäß Anspruch 5, worin das Mittel ferner 5-Fluorouracil oder ein Salz davon umfasst.

10. Verwendung von N-(3-Chloro-1H-indol-7-yl)-4-sulfamoylbenzolsulfonamid oder eines Salzes davon und Carboplatin oder eines Salzes davon zur Herstellung eines Mittels zur Vorbeugung oder zur Behandlung von Krebs, worin N-(3-Chloro-1H-indol-7-yl)-4-sulfamoylbenzolsulfonamid oder ein Salz davon und Carboplatin oder ein Salz davon gleichzeitig zu verabreichen sind.

11. Verwendung von N-(3-Chloro-1H-indol-7-yl)-4-sulfamoylbenzolsulfonamid oder eines Salzes davon und Carboplatin oder eines Salzes davon zur Herstellung eines Mittels zur Vorbeugung oder Behandlung von Krebs, worin N-(3-Chloro-1H-indol-7-yl)-4-sulfamoylbenzolsulfonamid oder ein Salz davon zu verabreichen ist und Carboplatin oder ein Salz davon nach einem vorbestimmten Zeitraum zu verabreichen ist.

12. Pharmazeutischer Kit für die gleichzeitige oder getrennte Verabreichung von wirksamen Mengen von N-(3-Chloro-1H-indol-7-yl)-4-sulfamoylbenzolsulfonamid oder eines Salzes davon und Oxaliplatin oder eines Salzes davon an einen Patienten.

13. Pharmazeutischer Kit gemäß Anspruch 12, welcher ferner 5-Fluorouracil oder ein Salz davon umfasst.

**Revendications**

1. Composition médicinale comprenant un N-(3-chloro-1H-indol-7-yl)-4-sulfamoylbenzènesulfonamide ou son sel, combiné avec au moins une substance choisie parmi (1) la carboplatine ; (2) l'oxaliplatine ; et (3) un sel des substances mentionnées (1) à (2).

2. Composition médicinale selon la revendication 1, qui comprend le N-(3-chloro-1H-indol-7-yl)-4-sulfamoylbenzènesulfonamide ou son sel, et la carboplatine ou son sel.

3. Composition médicinale selon la revendication 1, qui comprend le N-(3-chloro-1H-indol-7-yl)-4-sulfamoylbenzènesulfonamide ou son sel, et l'oxaliplatine ou son sel.

4. Composition médicinale selon la revendication 3, qui comprend de plus le 5-fluorouracile ou son sel.

5. Utilisation de N-(3-chloro-1H-indol-7-yl)-4-sulfamoylbenzènesulfonamide ou son sel, et l'oxaliplatine ou son sel, pour produire un agent destiné à prévenir ou traiter le cancer.

6. Utilisation selon la revendication 5, dans laquelle le N-(3-chloro-1H-indol-7-yl)-4-sulfamoylbenzènesulfonamide ou son sel et l'oxaliplatine ou son sel doivent être administrés simultanément.

7. Utilisation selon la revendication 5, dans laquelle le N-(3-chloro-1H-indol-7-yl)-4-sulfamoylbenzènesulfonamide ou son sel doit être administré et l'oxaliplatine ou son sel doit être administrée après un temps prédéterminé.

8. Utilisation selon la revendication 5, dans laquelle l'oxaliplatine ou son sel doit être administrée et le N-(3-chloro-1H-indol-7-yl)-4-sulfamoylbenzènesulfonamide ou son sel doit être administré après un temps prédéterminé.

9. Utilisation selon la revendication 5, dans laquelle l'agent comprend, de plus, le 5-fluorouracile ou son sel.

**10.** Utilisation de N-(3-chloro-1H-indol-7-yl)-4-sulfamoylbenzènesulfonamide ou son sel, et la carboplatine ou son sel, pour produire un agent destiné à prévenir ou traiter le cancer, dans lequel le N-(3-chloro-1H-indol-7-yl)-4-sulfamoylbenzènesulfonamide ou son sel et la carboplatine ou son sel doivent être administrés simultanément.

**11.** Utilisation de N-(3-chloro-1H-indol-7-yl)-4-sulfamoylbenzènesulfonamide ou son sel, et la carboplatine ou son sel, pour produire un agent destiné à prévenir ou à traiter le cancer, dans lequel le N-(3-chloro-1H-indol-7-yl)-4-sulfamoylbenzènesulfonamide ou son sel doit être administré et la carboplatine ou son sel doit être administrée après un temps prédéterminé.

**12.** Kit pharmaceutique pour administrer des quantités efficaces de N-(3-chloro-1H-indol-7-yl)-4-sulfamoylbenzènesulfonamide ou son sel, et l'oxaliplatine ou son sel, simultanément ou séparément à un patient.

**13.** Kit pharmaceutique selon la revendication 12, qui comprend, de plus, le 5-fluorouracile ou son sel.

Fig. 1

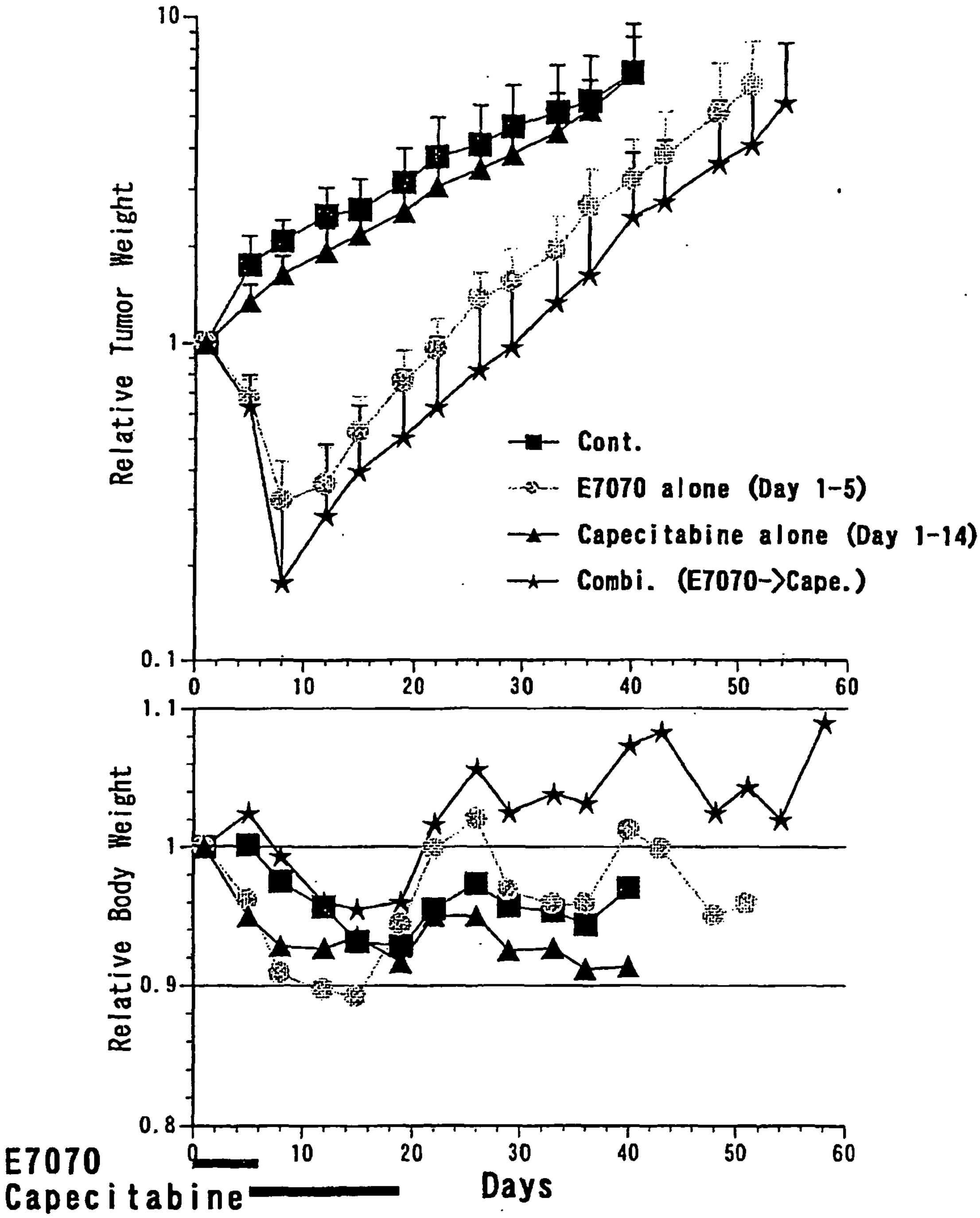

10
1
0.1
Relative Tumor Weight
Cont.
E7070 alone (Day 1-5)
Capecitabine alone (Day 1-14)
Combi. (E7070->Cape.)
0
10
20
30
40
50
60
1.1
1
0.9
0.8
Relative Body Weight
E7070
Capecitabine
Days

Fig. 2

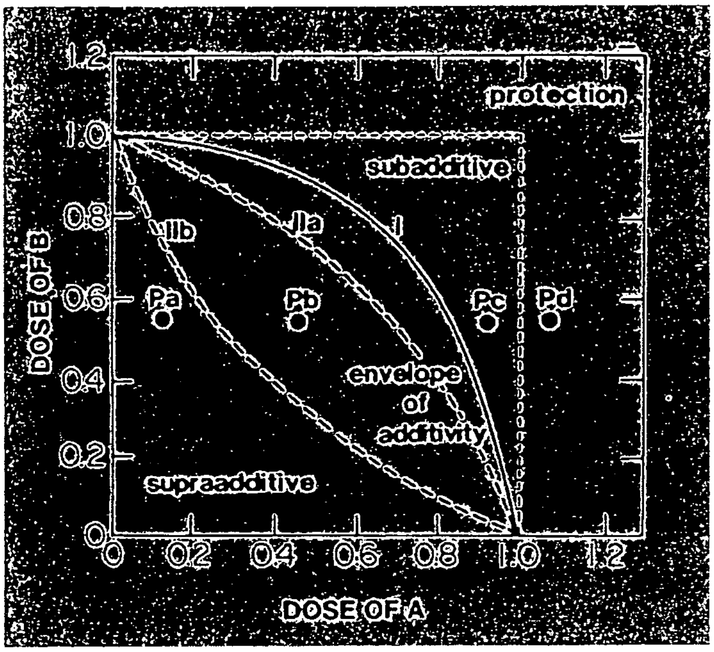
DOSE OF B
DOSE OF A
protection
subadditive
supraadditive
envelope of additivity
I
IIa
IIb
Pa
Pb
Pc
Pd
0
0.2
0.4
0.6
0.8
1.0
1.2

Fig. 3

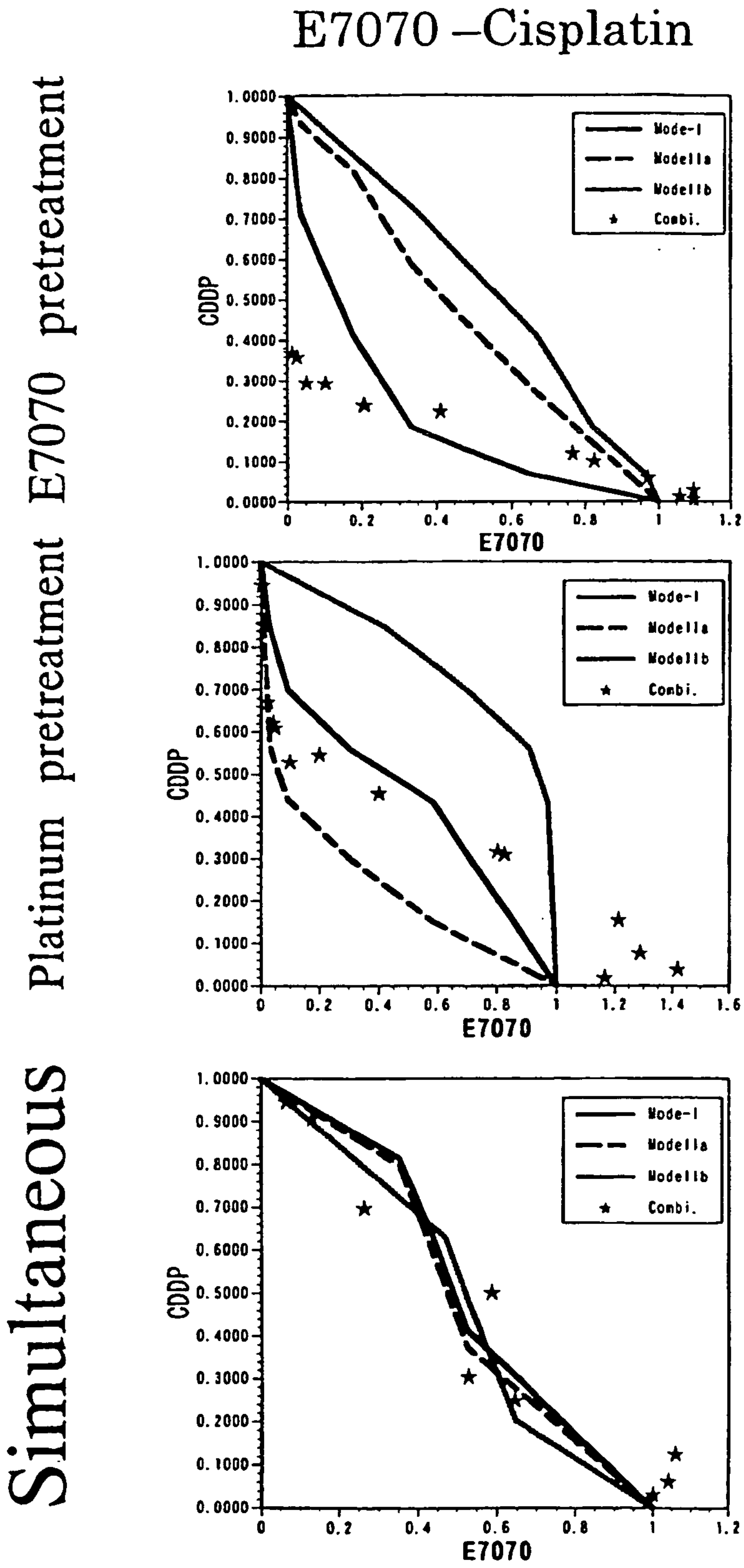
E7070 –Cisplatin
E7070 pretreatment
Platinum pretreatment
Simultaneous
CDDP
E7070
Mode-I
ModelIa
ModelIb
Combi.

Fig. 4

E7070 – Carboplatin

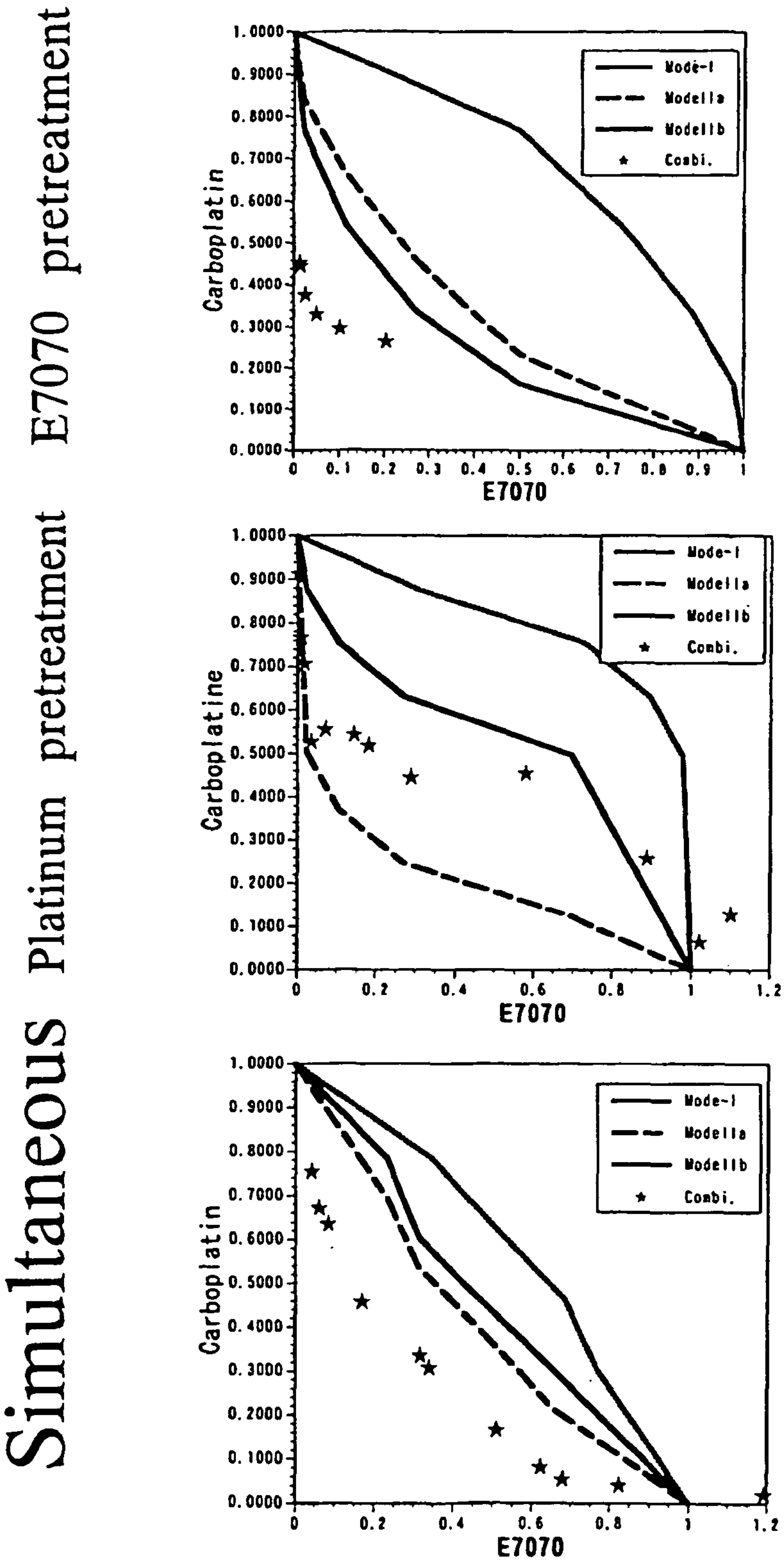

Fig. 5

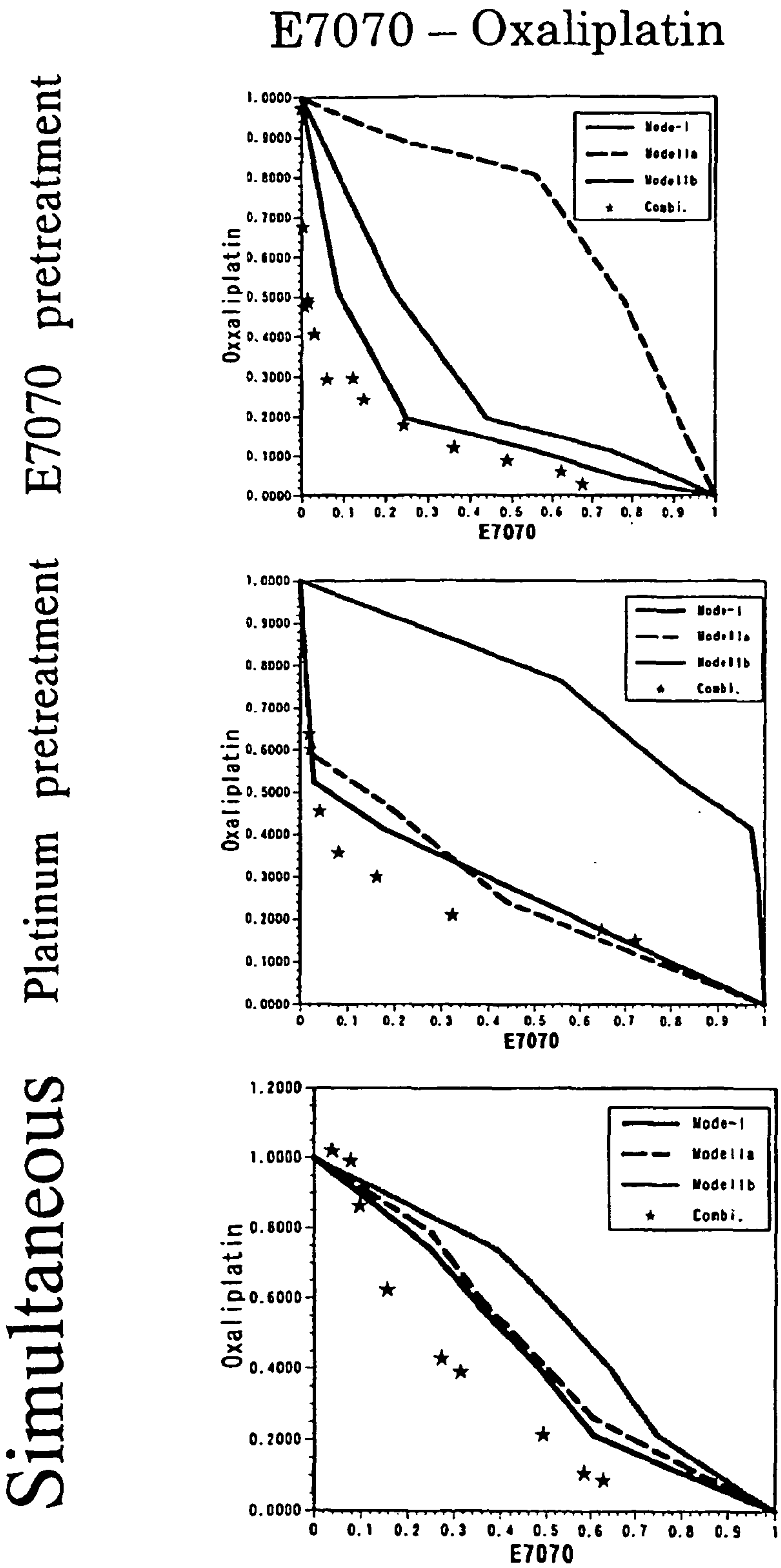
E7070 – Oxaliplatin
E7070 pretreatment
Platinum pretreatment
Simultaneous
Oxxaliplatin
Oxaliplatin
E7070
Mode-I
ModelIa
ModelIb
Combi.
1.0000
0.9000
0.8000
0.7000
0.6000
0.5000
0.4000
0.3000
0.2000
0.1000
0.0000
1.2000
0 0.1 0.2 0.3 0.4 0.5 0.6 0.7 0.8 0.9 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7165708 A
- WO 9507276 A


### Non-patent literature cited in the description


- *Cancer Chemother Pharmacol.,* 1992, vol. 30 (1), 25-30
- *J Natl Cancer Inst.,* 05 June 1996, vol. 88 (11), 747-53
- *Bone,* May 1999, vol. 24 (5), 491-7
- *Int J Radiat Oncol Biol Phys,* 1979, vol. 5, 85-91
- *Int J Cancer,* 1992, vol. 50, 604-610